# EUROPEAN PATENT SPECIFICATION

(11) **EP 0 897 922 B1**
(45) Date of publication and mention of the grant of the patent: **20.07.2005**
(21) Application number: 98202861.5
(22) Date of filing: 13.11.1992
(51) Int. Cl.: C07D 451/02, A61K 51/00, A61K 51/04

(54) **Cocaine receptor binding ligands**
Kokainrezeptor bindende Liganden
Ligands liant les récepteurs de la cocaine

(30) Priority: 15.11.1991 US 792648
(43) Date of publication of application: 24.02.1999
(62) Divisional of application: 92924277.4
(73) Proprietor: RESEARCH TRIANGLE INSTITUTE, Research Triangle Park North Carolina 27709 (US); NATIONAL INSTITUTES OF HEALTH, Bethesda, MD 20892 (US)
(72) Inventor: Kuhar, Michael J, Baltimore, MD 21209 (US); Boja, John W, Baltimore, MD 21236 (US); Carrol, Franck Ivy, Durham, NC 27712 (US); Lewin, Anita H, Chapel Hill, NC 27515-3483 (US); Abraham, Philip, Cary, NC 27511 (US)
(74) Representative: Poulin, Gérard

(56) References cited:
- WO-A-92/02260
- F. IVY CARROLL ET AL.: "Synthesis, ligand binding, QSAR, and CoMFA study of 3 beta-(p-substituted phenyl)tropane-2-beta-carboxylic acid methyl esters" JOURNAL OF MEDICINAL CHEMISTRY., vol. 34, no. 9, - September 1991 pages 2719-2725, XP002086621 WASHINGTON US
- CHEMICAL ABSTRACTS, vol. 114, no. 7, 18 February 1991 Columbus, Ohio, US; abstract no. 62429c, NASEREE, T.M. ET AL.: "Synthesis of tritium-labeled (3H)WIN 35,065-2; a new radioligand for cocaine receptors." XP002086624 & J. LABELLED COMPD. RADIOPHARM., vol. 28, no. 9, - 1990 pages 1011-1016,
- JOHN L. NEUMEYER ET AL.: "(123 I)-2-beta-carbomethoxy-3beta-(4-iodophenyl )tropane: high-affinity SPECT radiotracer of monoamine reuptake sites in the brain" JOURNAL OF MEDICINAL CHEMISTRY., vol. 34, no. 10, - October 1991 pages 3144-3146, XP002086622 WASHINGTON US
- CLARKE R L ET AL: "COMPOUNDS AFFECTING THE CENTRAL NERVOUS SYSTEM 4.3BETA-PHENYLTROPANE-2-CARBOXYLIC ESTERS AND ANALOGS" JOURNAL OF MEDICINAL CHEMISTRY, vol. 16, no. 1, November 1973, pages 1260-1267, XP000645455
- FIVY CARROLL ET AL.: "Isopropyl and phenyl esters of 3beta-(4-substituted phenyl)tropan-2-beta-carboxylic acids.Potent selective compounds for the dopamine transporter" JOURNAL OF MEDICINAL CHEMISTRY., vol. 35, no. 13, - 26 June 1992 pages 2497-2500, XP002086623 WASHINGTON US

## Description

This invention is directed to a class of binding ligands for cocaine and other receptors in the brain. Specifically, a novel family of compounds shows high binding specificity and activity, and, in a radiolabeled form, can be used to bind to these receptors, for biochemical assays and imaging techniques.

### Background Art

This application claims priority from US-A-5 128 118 and WO 92/02260. In US-A-5 128 118, there is disclosure of a family of compounds exhibiting particularly high specificity and affinity for cocaine receptors and other neurotransmitter receptors in the brain of the formula A. Wherein the broken line represents an optional chemical bond and the substituents at 2 and 3 may be at any position;

The iodo substituent may be at o, m, p, or multisubstituted;
R₁ = CH₃, CH₂CH=CH₂, (CH₂)ₙC₆H₅ n = 1-4;
R₂ = CH₃, C₂H₅, CH₃(CH₂)₃, (CH₃)₂CH, C₆H₅, C₆H₅CH₂, C₆H₅(CH₂)₂;
X = pharmacologically acceptable anion 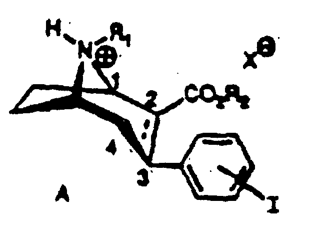
Sites of specific interest included cocaine receptors associated with dopamine transporter sites.

Subsequently, in the U.S. PCT Application from which priority is claimed the values for R₁ and R₂ were expanded, such that R₁ may be an alkyl of 1-7 carbon atoms, CH₂CR₃=CR₄R₅ wherein R₃-R₅ are each, independently C₁₋₆ alkyl, or phenyl compounds of the formula C₆H₅(CH₂)_{y}, wherein y = 1-6. The PCT filing also reveals the affinity of these compounds for cocaine receptors associated with serotonin transporters, and confirms, for the first time, that the in vitro binding reported in the earlier-filed application, is confirmed in in vivo testing. Specific disclosure for a variety of applications, including using the receptors in both PET and SPECT scanning, wherein either the iodine substituent, or one of the carbon groups is radioactive (I-123, 125 or 131 and C11) thus providing methods for scanning for the presence of specific cocaine receptors appears. Such scanning processes may be used to determine physiological conditions, such as Parkinson's Disease, to examine in general the density and distribution of specific cocaine receptors in various parts of the brain and/or body, to determine the efficacy of neurological treatments aimed at halting or reversing the degeneration of specific nerves in the brain, and screening drugs, such as antidepressant drugs.

The affinity of these compounds, as reported in the applications incorporated, is surprisingly high, and compared with prior art compounds, such as [³H]WIN 35,428, the novel compounds of these applications exhibit extremely low IC₅₀ values for binding inhibition.

Other compounds exhibiting this type of binding activity and specificity would further advance this art, making it possible to obtain better scanning, with higher reliability, and lower levels.

### Disclosure of the Invention

Applicants' invention resides in the discovery of a new family of compounds of the formula 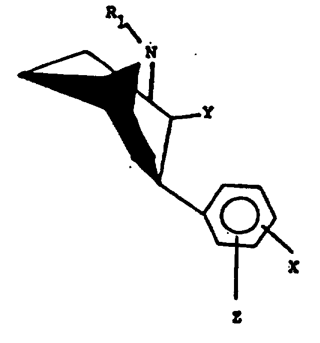 Wherein Y = CO₂R₂
- R₁ =: hydrogen, C₁₋₅ alkyl,
- R₂ =: hydrogen, C₁₋₆ alkyl, C₃₋₈ cycloalkyl, C₁₋₄ alkoxy, C₁₋₆ alkynyl, halogen or amine,

- X =: C₁₋₆ alkyl, C₃₋₈ cycloalkyl, C₁₋₄ alkoxy, C₁₋₆ alkynyl, halogen, amino, acylamido,
and
- Z =: I, Br, Cl, F, CN, CF₃ NO₂, N₃, OR₁, CO₂NH₂, CO₂R₁, C₁₋₆ alkyl, NR₄R₅, NHCOR₅, NHCO₂R₆,
wherein R₄-R₆ are each are each C₁₋₆ alkyl and wherein said compound lears at least one iodine or carbon atom which is radioactive.

These compounds exhibit usually high affinity for binding to receptors for the dopamine transporter site, as well as the serotonin transporter site, again based on inhibition of [³H]WIN 35,428 binding. It will be immediately apparent that certain of the compounds embraced within this novel class exhibit iodine substituents, and thus can be made easily radioactive, by substitution of a radioactive iodine, according to the synthesis scheme set forth in the prior applications. In those circumstances where no radioactive iodine is provided, the compounds may be made radioactive by selective use of at least one carbon that is radioactive, e.g., [¹¹C].

### Best Mode for Carrying Out the Invention

The compounds of this invention can be prepared according to the synthesis methods described in the parent applications. Additionally, specific synthesis routes and examples are set forth herein.

3β-[3'-Iodo-4'-aminophenyl]tropan-2β-carboxylic Acid Methyl Ester Dihydrochloride (1a). To a solution of 3β-[4'-aminophenyl]tropan-2β-carboxylic acid methyl ester (300 mg, 1.094 mmol) in glacial AcOH (15 mL) was added dropwise ICI (195 mg, 1.201 mmol) at room temperature for 3 h under N₂. After removal of solvent, the residue was diluted with H₂O, and then the pH was adjusted to basic with concentrated ammonia solution. The mixture was extracted with CHCl₃ which was washed with water and brine. After drying over MgSO₄, the solvent was evaporated to an oily product which was purified by flash chromatography (hexane-ether, 4:1). The collected fraction was converted to HCl salt with HCl/ether to yield 140 mg (29%) of 3β-[3'-iodo-4'-aminophenyl]tropan-2β-carboxylic acid methyl ester dihydrochloride (1a): mp 170-173°C; [α]²⁵-90.9° (c 0.055, MeOH), ¹H NMR (250 MHZ, CDCl₃) δ1.65 (m, 3), 2.09 (m, 2), 2.2 (s, 3, NCH₃). 2.45 (m, 1), 2.75 (m, 1, H-2), 2.8 (m, 1, H-3), 3.33 (m, 1, H-5), 3.45 (m, 4, H-1, OCH₃), 3.95 (m, 2, NH₂), 6.65 (d, 1, J = 8.7, ArH, 7.05 (dd, 1, j = 8.7, J = 1.5, ArH), 7.42 (d, J = 1.5, 1, ArH).

Anal. Calcd for C₁₆H₂₁IN₂O₂·2 HCL·H₂O: C, 39.12; H, 5.13; N, 5.70. Found: C, 39.12, H, 5.16; N, 5.63.

3β-[3'-Iodo-4'-azidophenyl]tropan-2β-carboxylic Acid Methyl Ester Hydrochloride (1b). To a solution of 3,β-[3'-iodo-4'-aminophenyl]tropan-2β-carboxylic acid methyl ester dihydrochloride (1a) (90 mg, 0.1902 mmol) in 1 mL of AcOH (3M) was added an aqueous solution of NaNO₂ (17.3 mg, 0.2661 mmol, in 0.5 mL of H₂O) at 0°C. After 30 min at this temperature NaN₃ (19 mg, 0.2754 mmol) in 0.5 mL of H₂O was added dropwise to the reaction mixture and stirred for 30 min at 0°C, then 30 min at room temperature. After removal of all solvent by evaporation, the residue was dissolved in CHCl₃ and washed with H₂O. The organic layer was dried over MgSO₄ and concentrated to give an oil which was converted to HCl salt to yield 64 mg (72.7%) of 3β-[3'-iodo-4'-azidophenyl]tropan-2β-carboxylic acid methyl ester hydrochloride (1b) as a yellowish solid: mp 140-143°C; [α]²⁵-97.4° (c 0.115), MeOh); ¹H NMR (250 MHz, CDCl₃) δ 1.51-1.73 (m, 3) 2.07-2.16 (m, 2), 2.19 (s, 3, NCH₃) 2.47 (m, 1), 2.80-2.93 (m, 2), 3.32 (m, 1, H-5), 3.51 (s, 3, OCH₃), 3.54 (m, 1, H-1), 7.01 (d, 1, J - 7.7, ArH), 7.28 (dd, 1, J = 7.77, J = 1, ArH), 7.60 (d, 1, J = 1, ArH).

Anal. Calcd for C₁₆H₁₉IN₄O₂·HCl·H₂O: C, 39.98; H, 4.61; N, 11.65. Found: C, 39.96.

Alternative synthesis for related compounds will be apparent to those of ordinary skill in the art. Additional schemes follow hereinbelow.

### Synthesis

Treatment of 3β-(4-aminophenyl)tropan-2β-carboxylic acid methyl ester (1) with the appropriate halogen gives 2. Diazotization of 2 followed by the addition of sodium azide provides the 3-halo-4-azido analog 3 (Scheme 1).

Condensation of anhydroecgonine methyl ester (4) with the appropriate acylamide oxime gives the oxadiazole 5. Addition of the appropriate aryl lithium to 5 gives the cocaine analog 6. The addition of the appropriate aryl magnesium halide to 4 gives the analog 7 (Scheme 2).

Hydrolysis of 8 gives the acid 9. Reduction of 9 with diborane gives 10. Treatment of 9 with thionyl chloride, followed by the appropriate amine gives 11. Treatment of 10 with thionyl halide or acylating agent gives 12 and 13, respectively (Scheme 3).

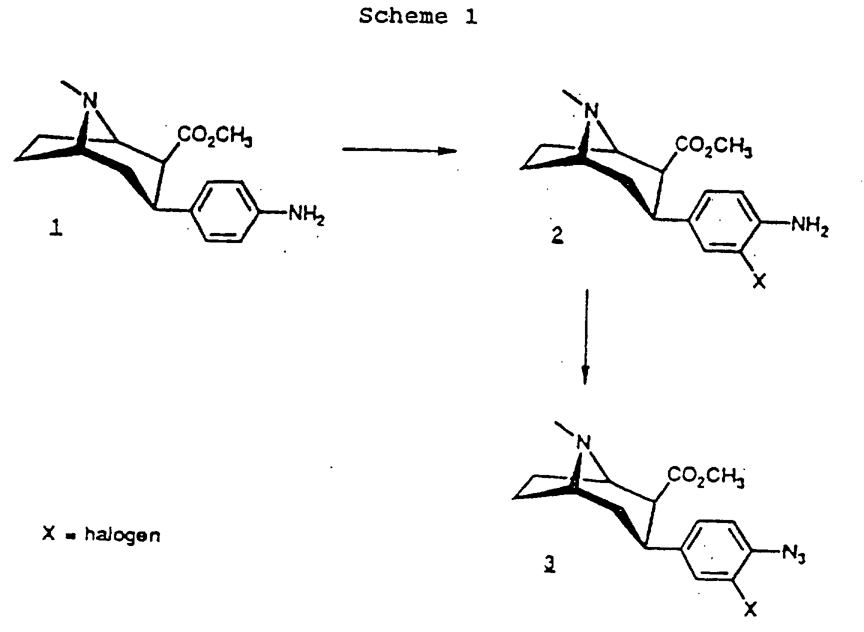

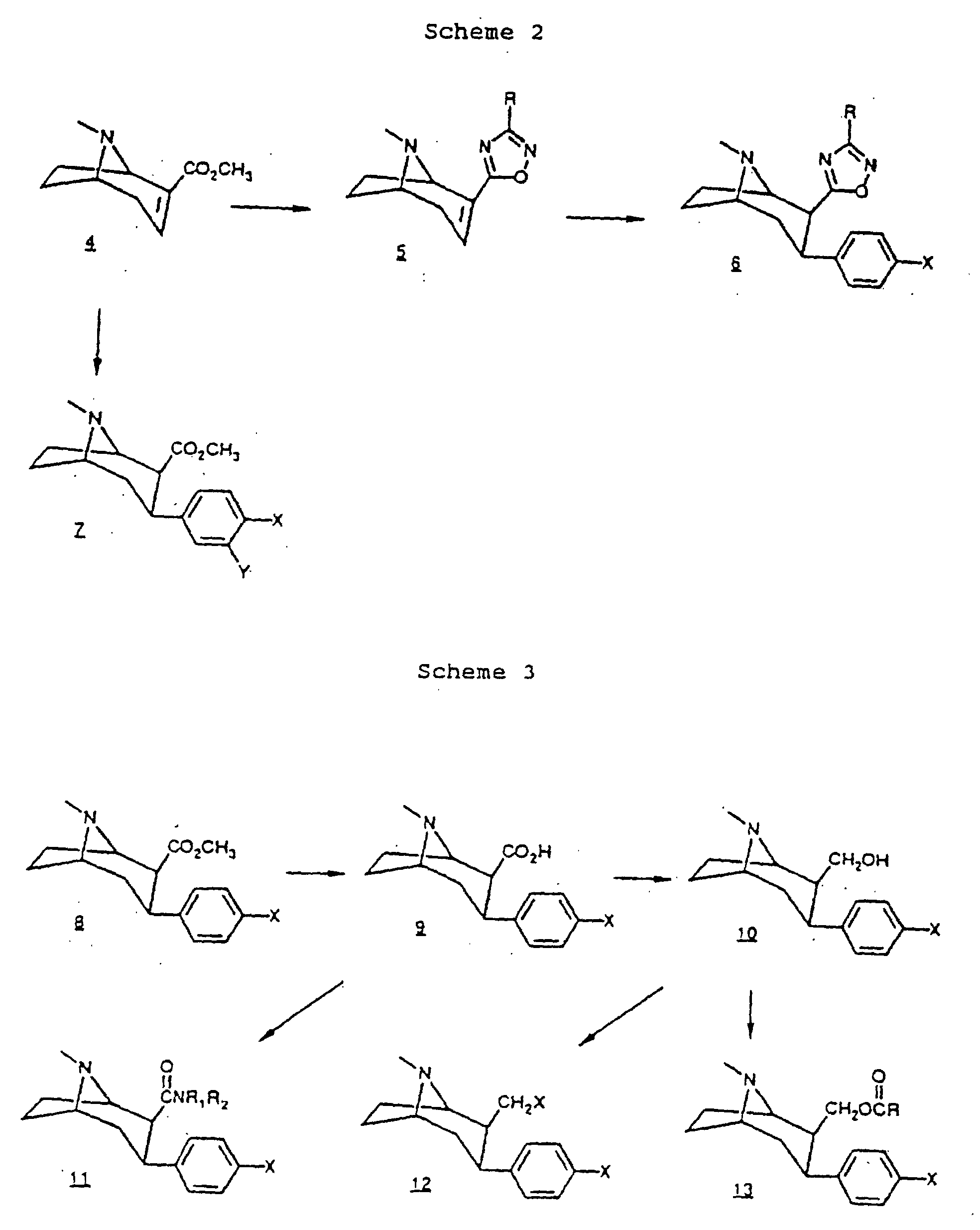

### Experimental

### 3β-(3-Bromo-4-aminophenyl)tropane Carboxylic Acid Methyl Ester (3, X = Br)

To a round-bottomed flask containing N,N-dimethylformamide (2.5 mL) was added 3β-(4-aminophenyl)tropane carboxylic acid methyl ester (100 mg, 0.365 mmol) and N-bromosuccinimide (64.5 mg, 0.365 mmol) under a stream of nitrogen gas at ambient temperature. The resulting solution immediately turned deep red. After stirring for an additional 2.5 h, water (5 mL) was added, and the crude reaction mixture was extracted with chloroform (3 x 25 mL). The combined organic extract was dried (MgSO₄) and concentrated under reduced pressure to yield the product as a brown oil. Flash chromatography (5% methanol-chloroform) afforded 42 mg (33%) of pure product as a yellow oil: mp of HCl salt 194°C dec; [α]_{D} -87.7° (c 0.09, MeOH); ¹H NMR (250 MHz, DMSO) δ 2.51-2.38 (m, 4), 3.39 (s, 3), 3.66-3.77 (td, 2, J = 12.5, 2.9 Hz), 4.17-4.59 (br s, 2), 4.67 (s, 3), 4.69-4.96 (br s, 2), 5.92 (br s, 2), 7.96-8.68 (m, 3H).

Anal. Calcd for C₁₆H₂₁BrN₂O₂ · 2 HCl · 2 H₂O): C, H, N.

### General Procedure for Hydrolysis of 3β-[4-Halophenyl]tropan-2β-carboxylic Acid Methyl Esters

The methyl ester (1.0 mmol) was dissolved in 20 mL 50% aqueous dioxane and heated to reflux. After 6 h, the solvent was evaporated and the residue crystallized from MeOH-Et₂O except as noted.

### 3β-(3,4-Dichlorophenyl)-2β-chloromethyltropane (7, X = Y = Cl)

To a three-neck, round-bottomed flask containing freshly distilled ether (125 mL) and magnesium turnings (268 mg, 11.0 mmol) was added 3,4-dichloroiodobenzene (2.26 g, 8.27 mmol). After 2 h, the reaction flask was equipped with a mechanical stirrer, and the Grignard reagent was cooled to -55°C before adding anhydroecgonine methyl ester (500 mg, 2.75 mmol). The resulting solution was stirred for an additional 2.5 h before being cooled to -78°C. After 1 h, 2 mL of trifluoroacetic acid was added to the solution followed by 2 h of stirring. The quenched reaction mixture was then diluted with 1 N HCl (100 mL) and extracted with ether (3 x 100 mL). The ethereal layers were discarded, and the aqueous layer was basified with conc. ammonium hydride and then extracted with chloroform (3 x 50 mL). The combined organic layers were dried (MgSO₄) and concentrated under reduced pressure to yield the crude product as a colorless oil. Flash chromatography (ether-triethylamine, 9:1) yielded 71 mg (9.0%) of pure product: ¹H NMR (250 MHz, CDCl₃) δ 1.52-1.76 (m, 2), 1.81-1.95 (m, 2), 1.96-2.22 (m, 2), 2.38 (s, 3), 3.07-3.15 (br s, 2), 3.21-3.32 (br s, 1), 3.45-3.65 (m, 1), 3.50 (s, 3), 7.10-7.38 (m, 3).

Anal. Calcd for C₁₆H₁₉Cl₂NO₂ · HCl): C, H, N.

### 3β-(4-Chloro-3-methylphenyl)-2β-chloromethyltropane (7, X = Cl, Y = CH₃)

To a three-neck, round-bottomed flask containing freshly distilled ether (125 mL) and magnesium turnings (200 mg, 8.25 mmol) was added 4-chloro-3-methylbromobenzene (1.69 g, 8.25 mmol). After 2 h, the reaction flask was equipped with a mechanical stirrer, and the Grignard reagent was cooled to -55°C before adding anhydroecgonine methyl ester (500 mg, 2.75 mmol). The resulting solution was stirred for an additional 2.5 h before being cooled to -78°C. After 1 h, 2 mL of trifluoroacetic acid was added to the solution followed by 2 h of stirring. The quenched reaction mixture was then diluted with of 1 N HCl (100 mL) and washed with ether (3 x 100 mL). The aqueous layer was basified with conc. ammonium hydroxide and extracted with CHCl₃ (3 x 50 mL). The combined organic layers were dried (MgSO₄) and concentrated under reduced pressure to yield the crude product as a colorless oil. Flash chromatography (ether-triethylamine, 9:1) yielded 45 mg (5.0%) of pure product: ¹H NMR (250 MHz, CDCl₃) δ 1.51-1.83 (m, 2), 1.97-2.21 (m, 2), 2.20 (s, 3), 2.45-2.59 (td, 1, J = 9.5, 2.6 Hz, 2.82-3.02 (m, 3), 3.34-3.40 (br s, 1), 3.51 (s, 3), 3.52-3.61 (br s, 1), 7.00-7.23 (m, 3)

Anal. Calcd for C₁₇H₂₂ClNO₂ · HCl · 2 H₂O: C, H, N.

### 3β-(3'-Methyl-4'-fluorophenyl)tropan-2β-carboxylic Acid Methyl Ester (7, X = F, Y = CH₃)

The title compound was prepared by modification of a reported procedure used to prepare other similar compounds.^{ref} Thus, using anhydroecgonine methyl ester (500 mg, 2.76 mmol) and 3-methyl-4-fluorophenyl magnesium bromide (prepared from 200 mg of magnesium metal and 1 mL of 3-methyl-4-fluoro-1-bromobenzene) yielded 234 mg (29%) of the title compound. The hydrochloride salt had mp 163-165°C; 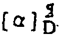 -103.8° (c 0.08, MeOH); ¹H NMR of free base of 41 (250 MHz, CDCl₃) δ 1.67 (m, 3), 2.15 (m, 2), 2.19 (s, 3, CH₃), 2.20 (s, 3, NCH₃), 2.55 (m, 2), 2.87 (m, 1, H-2), 2.93 (m, 1, H-3), 3.35 (m, 1, H-5), 3.49 (s, 3, OCH₃), 3.55 (m, 1, H-1), 6.85, 6.97 (m, 3, C₆H₃)

Anal. Calcd for C₁₇H₂₃ClFNO₂ · 1.5 H₂O: C, 57.54; H, 7.39; N, 3.95. Found: C, 57.88; H, 7.21; N, 4.20.

### [³H]WIN 35,428 Radioligand Binding

Rat striata from male Sprague-Dawley rats (250-350 g) were rapidly dissected, frozen, and stored at -70°C until used. The frozen rat striata were homogenized in 20 volumes of 10 mM phosphate buffer (pH 7.4) containing 0.32M sucrose using a polytron (setting 6) for 10 sec. The homogenate was centrifuged for 10 min at 50,000 x g, the resulting pellet was washed in buffer, recentrifuged, and resuspended to a tissue concentration of 10.0 mg/mL. Binding assays were carried out in a total volume of 0.5 mL containing 0.5 nM [³H]WIN 35,428 and 1.0 mg tissue. The suspensions were incubated for 2 h on ice. Incubations were terminated by filtration with three 5 mL washes through Whatman GF/B filters previously soaked in 0.05% polyethylenimine using a Brandel M48R filtering manifold (Brandel Instruments, Gaithersburg, MD). Radioactivity was counted in 5 mL of scintillation cocktail in a Beckman LS 3801 liquid scintillation counter with an efficiency of approximately 50%. Nonspecific binding of [³H]WIN 35,428 was defined by the presence of 30 µM (-)-Cocaine. Under these conditions, nonspecific binding was approximately 5-8% of total binding. IC₅₀ values were determined from competition curves of 10-12 points utilizing the curve fitting program EBDA. Mean values and standard errors were calculated from 3-4 assays for each test drug.

### Tissue Preincubation with Irreversible Agents

Tissue was prepared as described above, and the final homogenate was incubated for 60 min with either drug or vehicle as control for 60 min on ice in the above buffer. Following the 60 min incubation period, all compounds containing an azido group were then exposed to UV light (2800 Å) for 40 sec. The incubation of all compounds was terminated by centrifugation at 50,000 x g for 10 min. The resulting pellet was resuspended to a concentration of 10 mg/mL, and an aliquot was removed (0 washes). This procedure was repeated for a total of 3 washes. Residual [³H]WIN 35,428 binding was determined as described above. Data are expressed as the percent of specific control binding.

Testing of various compounds within the described class has given remarkably high binding values. Thus, as reported in the parent applications, receptor binding activity can be determined by degree of inhibition of the binding of [³H]WIN 35,428. In such assays, the ligand of interest is assigned a IC₅₀ value, when incubated in a 10 nM phosphate buffer, pH 7.4, containing 0.32^{■} sucrose, with 0.5 nM [³H]WIN 35,428 for a two hour period of time. After that, the radioactivity bound to the substrate is measured. As reported in US-A-5 128 118, on binding to a dopamine transporter receptor site, cocaine gave a IC₅₀ of 89.00 nM, WIN 35,428 gave a value of 14.00 nM and a compound representative of the subject matter claimed in that application, 3β-[4-iodophenyl]tropane-2β-carboxylic acid methyl ester tartrate gave a IC₅₀ value of 0.25 nM. In similar assays, values of 1.35 and 4.93 nM were obtained for compounds within the class set forth above, particularly, those compounds bearing a carboxylic acid moiety, or a heterocyclic moiety. Similar values may be obtained for the remaining members of the class.

When bearing an appropriate radioactive label, such as ¹¹C or ¹²³I, ¹²⁵I or ¹³¹I, these compounds, preferential binders to dopamine transporter and serotonin transporter binding sites, can be used as imaging agents for both positron emission tomography (PET) as well as single photon emission computed tomography (SPECT). PET may require the [¹¹C] labeled form of the drug, while radioactive iodine-labeled compounds may be used in SPECT scanning.

As noted, such scanning has a variety of utilities. The actual density and distribution of cocaine receptors in various parts of the brain and CNS is of interest, and can be mapped through the use of these compounds. Additionally, as noted above, identification of degeneration of nerve terminals, corresponding to a loss of dopamine transporter sites, can be determined by this scanning, to diagnose Parkinson's Disease. In addition, progression of the disease, and efficacy of treatment, can be monitored by such scanning. Similarly, degeneration of specific nerves in the brain due to exposure to various toxins can be detected or monitored by the scanning made possible by these compounds.

As an additional use, drugs having high affinity for the transporter sites bound to by these compounds, particularly serotonin and dopamine transporter sites, can be screened, using these compounds, in the identified scanning methods. The scanning itself is conventional, given the use of these compounds, and does not constitute an aspect of the invention per se. Affinity values for representative compounds are given in the following table.

**Table**

| Potencies of Cocaine and Analogs in Inhibiting Binding of [³]-3β-(4-Fluorophenyl)tropan-2β-carboxylic Acid Methyl Ester (WIN 35,428) | |
|---|---|
| Compound | IC₅₀ (nM) |
| Cocaine | 102 |
| 2 (X = I) | 1.35 |
| 3 (X = I) | 4.93 |
| 7 (X = Y = Cl) | 0.79 |
| 7 (X = Cl, Y = CH₃) | 0.81 |

This invention has been described in both generic terms, and by reference to specific description.

## Claims

1. A compound of the formula below, 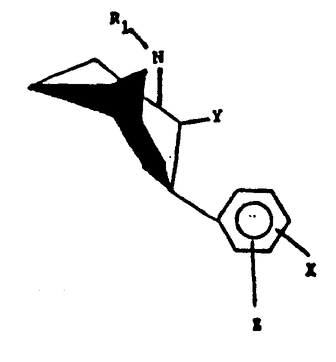 wherein Y = CO₂R₂
R₁ = hydrogen, C₁₋₅ alkyl,
R₂ = hydrogen, C₁₋₆ alkyl, C₃₋₈ cycloalkyl, C₁₋₄ alkoxy, C₁₋₆ alkynyl, halogen or amine,
X = C₁₋₆ alkyl, C₃₋₈ cycloalkyl, C₁₋₄ alkoxy, C₁₋₆ alkynyl, halogen, amino, acylamido,
and
Z = I, Br, Cl, F, CN, CF₃ NO₂, N₃, OR₁, CO₂NH₂, CO₂R₁, C₁₋₆ alkyl, NR₄R₅, NHCOR₅, NHCO₂R₆,
wherein R₄-R₆ are each C₁₋₆ alkyl and wherein said compound bears at least one iodine or carbon atom which is radioactive.

2. The compound of claim 1, wherein X and Z are Cl.

3. The compound of claim 1, wherein X is Cl and Z is CH₃.

4. The compound of anyone of claims 1 to 3, wherein said radioactive atom is [¹¹C].

5. The compound of anyone of claims 1 to 3, wherein said compound bears an iodine atom, and said iodine atom is radioactive, and selected from the group consisting of ¹²³I, ¹²⁵I and ¹³¹I.

## Patentansprüche

1. Verbindung der nachfolgenden Formel 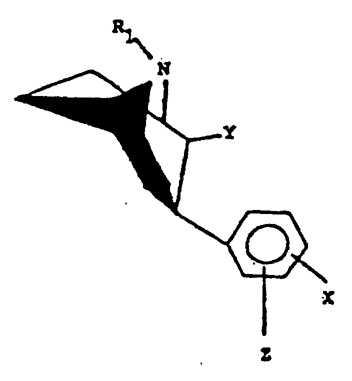 worin Y = CO₂R₂,
R₁ = Wasserstoff, C₁₋₅-Alkyl,
R₂ = Wasserstoff, C₁₋₆-Alkyl, C₃₋₈-Cycloalkyl, C₁₋₄-Alkoxy, C₁₋₆-Alkinyl, Halogen oder Amin,
X = C₁₋₆-Alkyl, C₃₋₈-Cycloalkyl, C₁₋₄-Alkoxy, C₁₋₆-Alkinyl, Halogen, Amino, Acylamido, und
Z = I, Br, Cl, F, CN, CF₃, NO₂, N₃, OR₁, CO₂NH₂, CO₂R₁, C₁₋₆-Alkyl, NR₄R₅, NHCOR₅, NHCO₂R₆,
wobei R₄-R₆ jeweils C₁₋₆-Alkyl sind und wobei die Verbindung wenigstens ein Iod- oder Kohlenstoffatom trägt, das radioaktiv ist.

2. Verbindung nach Anspruch 1, worin X und Z Cl sind.

3. Verbindung nach Anspruch 1, worin X Cl ist und Z CH₃ ist.

4. Verbindung nach irgendeinem der Ansprüche 1 bis 3, wobei das radioaktive Atom [¹¹C] ist.

5. Verbindung nach irgendeinem der Ansprüche 1 bis 3, wobei die Verbindung ein Iodatom trägt und dieses Iodatom radioaktiv und aus der Gruppe ausgewählt ist, bestehend aus ¹²³I, ¹²⁵I und ¹³¹I.

## Revendications

1. Composé de la formule ci-après, 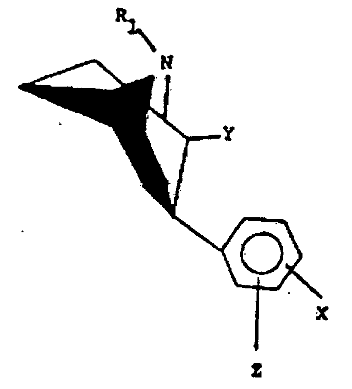 dans laquelle Y = CO₂R₂
R₁ =hydrogène, alkyle en C₁ à C₅,
R₂ =hydrogène, alkyle en C₁ à C₆, cycloalkyle en C₃ à C₈, alcoxy en C₁ à C₄, alcynyle en C₁ à C₆, halogène ou amine,
X = alkyle en C₁ à C₆, cycloalkyle en C₃ à C₈, alcoxy en C₁ à C₄, alcynyle en C₁ à C₆, halogène, amino, acylamido, et
Z = I, Br, Cl, F, CN, CF₃, NO₂, N₃, OR₁, CO₂NH₂, CO₂R₁, alkyle en C₁ à C₆, NR₄R₅, NHCOR₅, NHCO₂R₆,
où R₄ à R₆ sont chacun un alkyle en C₁ à C₆,
et ledit composé portant au moins un atome d'iode ou de carbone qui est radioactif.

2. Composé selon la revendication 1, dans lequel X et Z sont Cl.

3. Composé selon la revendication 1, dans lequel X est Cl et Z est CH₃.

4. Composé selon l'une quelconque des revendications 1 à 3, dans lequel ledit atome radioactif est du carbone-11.

5. Composé selon l'une quelconque des revendications 1 à 3, ledit composé portant un atome d'iode, et ledit atome d'iode étant radioactif et choisi dans le groupe constitué par l'iode-123, l'iode-125 et l'iode-131.
